(19) Europäisches Patentamt / European Patent Office / Office européen des brevets

(11) **EP 4 064 293 A1**

(12) **EUROPEAN PATENT APPLICATION**
published in accordance with Art. 153(4) EPC

(43) Date of publication:
**28.09.2022 Bulletin 2022/39**

(21) Application number: **20842588.4**

(22) Date of filing: **20.11.2020**

(51) International Patent Classification (IPC):
*G16H 50/20* (2018.01)      *A61B 5/00* (2006.01)
*G16H 10/60* (2018.01)      *G16H 50/70* (2018.01)

(52) Cooperative Patent Classification (CPC):
**G16H 50/20;** G16H 10/60; G16H 50/70

(86) International application number:
**PCT/ES2020/070723**

(87) International publication number:
**WO 2021/099669 (27.05.2021 Gazette 2021/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.11.2019 ES 201931028**

(71) Applicants:
• **Fundacio Salut del Consorci Sanitari del Maresme**
  **08304 Mataro (ES)**
• **Clave Civit, Pere**
  **08304 Barcelona (ES)**

• **Tibau Alberdi, Xavier**
  **08304 Barcelona (ES)**
• **Martin Martinez, Alberto**
  **08304 Barcelona (ES)**

(72) Inventors:
• **CLAVE CIVIT, Pere**
  **08304 Barcelona (ES)**
• **TIBAU ALBERDI, Xavier**
  **08304 Barcelona (ES)**
• **MARTIN MARTINEZ, Alberto**
  **08304 Barcelona (ES)**

(74) Representative: **Mohammadian, Dario KUKATI**
  **Apartado de Correos 34031**
  **08080 Barcelona (ES)**

(54) **SYSTEM AND METHOD FOR THE IMPROVED DIAGNOSIS OF OROPHARYNGEAL DYSPHAGIA**

(57)    Different aspects of the invention implement a system, and corresponding method, for the systematic, universal and optimized screening of oropharyngeal dysphagia which is based on an algorithm which takes into account parameters and clinical record of each patient, for determining with high probability the possibilities of suffering from oropharyngeal dysphagia, and selecting only those patients which really have a risk of suffering from oropharyngeal dysphagia for the continuation of their medical diagnosis and clinical exploration and instrumental assessment phases.

FIG. 2

**Description**

**TECHNICAL FIELD**

The invention refers generally to the field of oropharyngeal dysphagia diagnostics, and, in particular, to a system and method for the optimized diagnosis of oropharyngeal dysphagia and swallowing disorders, including alterations in the safety (aspirations, penetrations) and efficacy of the swallowing (oropharyngeal residue).

**BACKGROUND ART**

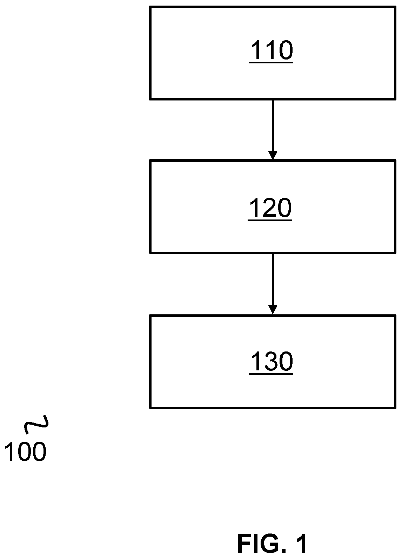

Traditionally, the diagnosis 100 of oropharyngeal dysphagia (from now on, OD) comprises three phases, the initial screening 110 (by means of a questionnaire or a clinical interview), clinical exploration 120 and instrumental evaluation 130, as represented in **FIG. 1**. Although dysphagia has been recently recognized as a geriatric syndrome and every time there are more health professionals dealing with its management, OD is an unknown and under-diagnosed pathology. It presents a high prevalence in many population groups which make frequent use of the hospital system (elderly, neurological, cognitive impairment, and so on), with high poly-morbidity (respiratory infections, malnutrition and dehydration) and mortality. Additionally, to the bad health results of the patients which present OD, they are re-admitted more and require more resources in order to guarantee the continuation of the assistance.

The first interview phase 110, or screening, is performed by means of interviewing the patient and/or caregivers and/or family (which depends on their degree of acquaintance and implication with the cares). The objective of interviewing the patient by the doctors, nurses, and other health professionals is detecting clinical indications and predisposition factors of a patient to have OD, and in which risk factors, clinical indicators of safety and efficacy and/or screening by means of validated instruments (EAT-10; TOR-BSST; Water Test; GUSS; MASA), are evaluated. Screening, or filtering, or early diagnosis, means selecting, via a method, those patients which present the highest risk of suffering from dysphagia and to whom a clinical or instrumental exploration must be performed in order to confirm the diagnosis. The filtering is usually performed using simple methods that do not need a large training of the personnel whilst the clinical and instrumental diagnosis does require highly trained and qualified health professionals. In the biomedical field, a clinical examination of a person refers to determining the presence or absence of a determined illness.

In parallel, other validated questionnaires exist which help in the filtering of dysphagia:

- MASA: Created by Mann for evaluating the difficulties while eating or swallowing. It is used in patients with stroke. It consists of 24 items that are scored between 5-10 each one, being 200 the maximum score (170-200 normal; 149-169 light; 141-148 moderate; < 141 severe).
- Toronto Bedside Swallowing Screening Test: Identifies the difficulty in the swallowing in patients with stroke. It is based in exploring the tongue's strength, and evaluating voice after each one of 10 swallows of 5ml that compose the test.
- Yale Swallow Protocol: Consists in evaluating the aspiration risk based on a few cognitive questions, examining the swallowing mechanism (labial seal, lingual function and facial symmetry). Also, 90cc of water are fed to the seated patient to drink continuously. If the patient coughs or chokes, the test is positive.
- Gugging Swallowing Screen: This method determines the aspiration risk in neurological patients. The test starts with swallowing saliva followed by swallowing semisolid, fluid, or solid, textures. GUSS comprises 4 subtests and is divided into 2 parts: the preliminary evaluation or the indirect swallowing test (subtest 1) and the direct swallowing test, which comprises 3 subtests. These 4 subtests are to be performed sequentially. In the indirect swallowing test, the following is evaluated: a) watchfulness; b) voluntary cough and/or throat clearing and c) saliva ingestion is evaluated (swallowing, drooling, voice change). The direct swallowing evaluates a) swallowing, b) involuntary cough, c) drooling and d) voice change in semisolid swallowing, liquid swallowing and the solid swallowing tests. Evaluation is based in a system of points, for each subtest, a maximum of 5 points can be attained. Twenty points are the highest score that a patient can reach, and denotes the normal swallowing capacity without aspiration risk. In total, 4 levels of severity can be determined: 0-9 points: serious dysphagia; 10-14 points: moderate dysphagia; 15-19 points: light dysphagia; 20 points: normal swallowing ability.

Depending on the results of the first screening phase, the second phase 120 of clinical exploration of swallowing, wherein the Volume-Viscosity Swallow Test, V-VSTV-VST, and in case necessary, the third phase 130 of instrumental evaluation, is performed by the specialized personnel. The sensibility of V-VSTV-VST in dysphagia diagnosis has a high Se (93%) and a high Sp (80%), jointly with a global reliability value Kappa of 0.77 (95%). The V-VST is a clinical test which uses different volumes (5, 10 and 20 mL) and viscosities (nectar, liquid and pudding) to detect signs of changes in the efficacy and safety of the swallowing. The purpose of V-VST is to identify the clinical signs of the alterations in the swallowing efficacy (labial seal, oropharyngeal residue, partitioned swallowing), and the clinical signs of the impairment in the swallowing safety, changes in voice quality, coughing, or decrease in oxygen saturation by 3 or more percentage points with respect to the basal saturation of the patient measured with a pulsimeter. The pulsimeter measures indirectly the oxygen saturation in blood of a patient, expressing the result in percentage. The cough and/or fall in oxygen saturation of 3 or more percentage points are considered clinical signs of tracheobronchial aspiration. Performing V-VST permits detecting people who suffer OD, as well as adapting their hydration by oral means, adjusting the volume and viscosity of the fluids to provide a safe swallowing for the patient. The care procedure can be performed at any moment.

The third phase 130 of instrumental evaluation is performed by means of Videofluoroscopy, VFS, and/or by means of fibrolaryngoscopy, FEES, (Fiberoptic endoscopic evaluation of swallowing), which has as a result determining with high probability the existence of OD in the patient. The instrumental methods provide a precise and objective diagnosis and they are the ideal diagnosis means for those patients who need a more precise evaluation. The VFS consists in a dynamic radiological exploration which determines the security and efficacy of the swallowing and additionally permits knowing the oropharyngeal motor response. VFS can determine if the aspiration are associated with an altered glosopalatine seal, a delay in the onset of pharyngeal swallowing or a deterioration in the protection of the respiratory ways (closure of the vocal chords), or an ineffective cleaning of the pharynx (post-swallowing aspiration). FEES permits "in situ" observing and video recording of the pharynx by means of fibroscopy at the moment of swallowing. Both techniques permit understanding the aspiration mechanisms, safety alterations and swallowing efficacy in each patient. Despite the fact that the consequences of not treating OD have been widely described (increase in respiratory infections and malnutrition) and hospitalizing a prevalent 47.5% population ≥ 70 years, the OD screening is only performed to 12% of cases. Of these, only 61% of the patients present OD. Not detecting the patients who suffer from the illness by the screening reflects a decrease in clinical and instrumental diagnoses. This supposes a diagnosis process with low probability of success and additionally wastes resources dedicated to healthcare.

The patients who do not require the third phase of instrumental evaluation for their diagnosis are a majority, as a clinical diagnosis of dysphagia can be established based on the clinical reevaluation and V-VST exploration of alterations in the safety and swallowing efficacy and providing a safe hydration to the patients by selecting the volume and optimum viscosity minimizing the risk of suffering aspiration. The sooner V-VST is applied, the sooner patients with OD can be helped, for premature detection and minimization of their bad health results, and resource consumption. The lack of awareness and lack of sensibilization of the health community about OD results in V-VST and instrumental evaluation being performed on only 1 out of 10 elderly hospitalized patients. This is due to the fact that the doctors and nurses do not focus their explorations and interviews to the detection of dysphagia. Having regard for the high prevalence of OD in elderly hospitalized patients described in the literature which have not undergone V-VST and instrumental evaluation, worsens the detection, treatment and health results of the patients with OD.

These three diagnosis phases of OD require an important work load by specialists, they are slow and expensive. Frequently, it is found that patients who give a positive result in the initial screening, and undergo the remaining phases, never suffered from OD, wasting human, time, and economic resources (false positives). On the other hand, if the initial interview is not detailed enough, many patients with OD are not detected and the continued diagnostics process is not approved (false negatives), resulting in a bad diagnosis process which does not help those patients who really suffer from OD, whilst the population of patients and corresponding treatment costs increase.

Therefore, the inventors have detected the need to improve the conventional diagnosis process optimizing it in order to reduce both the false positives as well as the false negatives. This has been attained by improving the systematic screening phase, making sure that an incrementally increasing number of patients with a real risk of suffering from OD are evaluated in posterior diagnosis phases. Also, existing methods consume too many computational resources, are slow, and do not have the required precision. Therefore, the need exists to solve in an effective manner the described problems.

## SUMMARY OF THE INVENTION

It is an object of the invention to provide solutions to the above-mentioned problems. In particular, it is an object of the invention to provide apparatus and methods for optimized OD screening.

This optimization of the method of OD diagnosis is based on an algorithm that takes into account parameters and the clinical record of the patients, in order to determine with high probability that a patient is suffering from OD, and approving for its diagnosis the last two phases of clinical exploration 120 and instrumental evaluation 130 only those patients who really have a high probability of suffering from OD. The resulting algorithm is more precise, and also consumes less computational resources.

It is therefore an object of the invention to provide a system for the optimized screening of oropharyngeal dysphagia.

It is another object of the invention to provide a method for the optimized screening of oropharyngeal dysphagia.

It is another object of the invention to provide a computer program, comprising instructions which, once executed on a processor, perform the steps of a method for the optimized screening of oropharyngeal dysphagia.

It is another object of the invention to provide a computer readable medium, comprising instructions which, once executed on a processor, perform the steps of a method for the optimized screening of oropharyngeal dysphagia.

The invention provides methods and devices that implement various aspects, embodiments, and features of the invention, and are implemented by various means. The various means may comprise, for example, hardware, software, firmware, or a combination thereof, and these techniques may be implemented in any single one, or combination of, the various means.

For a hardware implementation, the various means may comprise processing units implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof.

For a software implementation, the various means may comprise modules (for example, procedures, functions, and so on) that perform the functions described herein. The software codes may be stored in a memory unit and executed by a processor. The memory unit may be implemented within the processor or external to the processor.

Various aspects, configurations and embodiments of the invention are described. In particular the invention provides methods, apparatus, systems, processors, program codes, computer readable media, and other apparatuses and elements that implement various aspects, configurations and features of the invention, as described below.

## BRIEF DESCRIPTION OF THE DRAWING(S)

The features and advantages of the present invention will become more apparent from the detailed description set forth below when taken in conjunction with the drawings in which like reference characters identify corresponding elements in the different drawings. Corresponding elements may also be referenced using different characters.

**FIG. 1** shows the three main phases in the OD diagnosis process.

**FIG. 2** shows different aspects of the system of optimized OD diagnosis according to an embodiment of the invention.

**FIG. 3** shows a training server according to one aspect of the invention.

**FIG. 4** shows the training method according to one aspect of the invention.

**FIG. 5** shows the variable selection step according to one aspect of the invention.

**FIG. 6** shows the expert module training step according to one aspect of the invention.

**FIG. 7** shows the OD risk prediction step according to one aspect of the invention.

**FIG. 8** shows the application of the system, and corresponding method, of the invention, to the conventional diagnosis process of FIG. 1.

**FIG. 9** shows the OD risk prediction step according to the RBDADI model according to another aspect of the invention.

**FIG. 10** shows a representation of the RBDADI model in terms of the number of arcs according to one aspect of the invention.

**FIG. 11** shows a representation of the CODO model in terms of the number of arcs according to one aspect of the invention.

## DETAILED DESCRIPTION OF THE INVENTION

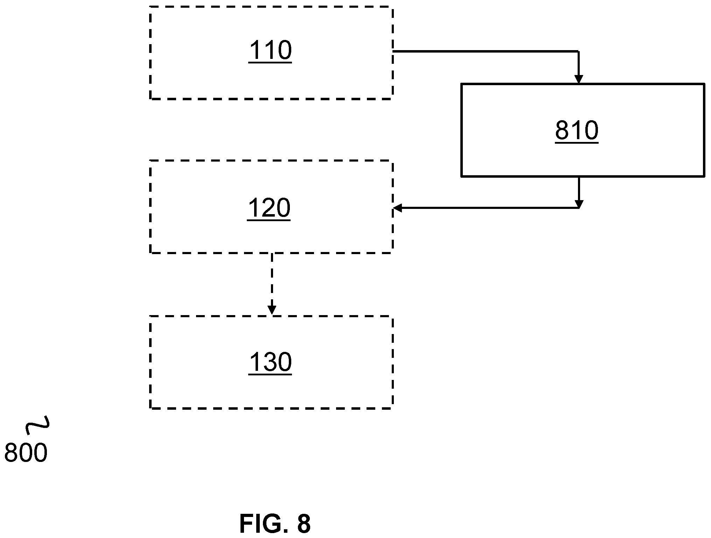

**FIG. 8** shows the application of the system, and corresponding method, of the invention, to the conventional diagnosis of FIG. 1, resulting in a system 800, and corresponding method, of optimized diagnosis including the systematic screening provided by the invention. The optimized diagnosis system 810, and corresponding method, is integrated between the first phase of initial screening 110 and the second phase of clinical exploration 120, to filter the number of patients allowed to proceed to the second 120 and third 130 phases of the diagnosis.

The objective of the diagnosis method steps is to filter the patients with a higher percentage risk of suffering from OD, so that the limited resources are invested in performing the V-VST clinical exploration to those patients with the highest risk of suffering from OD. In this manner, the sensibility of the clinical exploration is improved resulting in the identification of more patients that have high probabilities of suffering from OD. Many of these cases are currently not detected as there is no systematic screening and due to the lack of awareness and experience of the health professionals. Therefore, by using the diagnosis system and method, and knowing which patients have a higher risk, more OD cases can be detected using the same quantity of resources, therefore, improving the efficacy of this resource usage.

By means of an artificial intelligence tool, the patient type which presents a higher risk of suffering from oropharyngeal dysphagia is characterized and typified based on the digital registry of medical diagnoses, clinical characteristics and prescribed pharmaceutical products of its clinical record, for example, of the last two years, and the patient's risk of suffering from dysphagia is then established. The elderly patients who suffer from OD are frequently re-admitted to hospital, present a higher mortality, and consume large quantities of health resources as their morbidity increases with respect to patients with the same clinical conditions, but without OD. By means of the expert module, the most important diagnosis codes for the detection of dysphagia are identified, which are used to establish the risk of suffering from dysphagia by a patient, and all without any diagnosis tests nor other test to perform on the user. This last point is quite important as it permits performing public health campaigns, and, for example, notify health system users identified with a risk of suffering from dysphagia, that they should perform more specific tests.

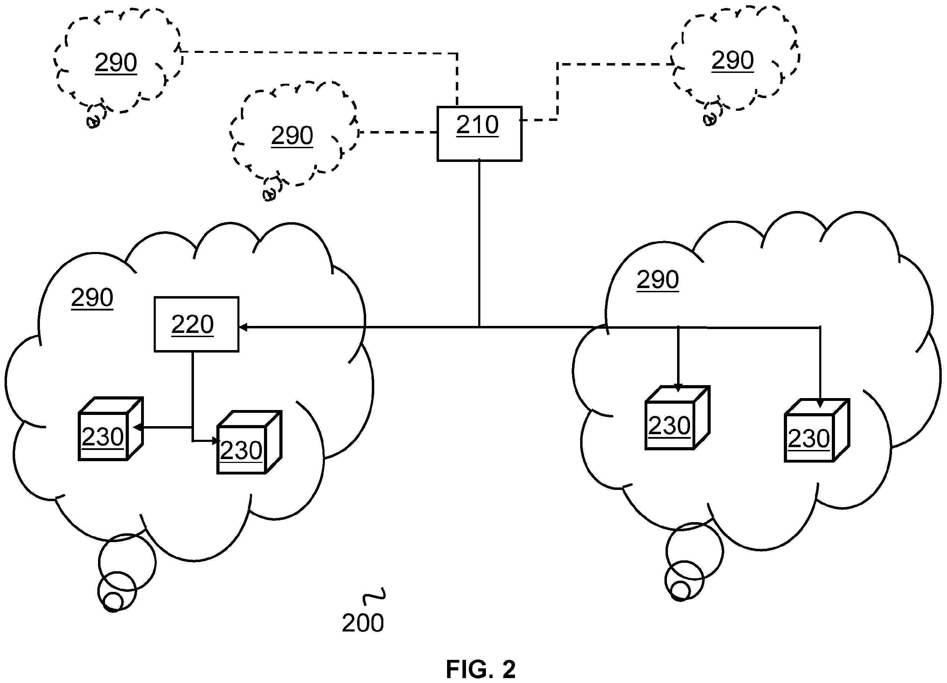

FIG. 2 shows in improved OD screening system according to one embodiment of the invention. In one aspect of the embodiment (the population 290 to the left of the figure), the system 200 comprises at least one training server 210 which is based on an artificial intelligence algorithm, at least one OD prediction server 220, and at least one patient query terminal 230. The query terminals serve the purpose to launch an OD diagnosis query about the patient by transmitting a communication to the corresponding prediction server. In the meantime, the prediction server has obtained the latest update from the expert module of the training server and determines the risk from suffering from OD using this model and the patient data received from the query terminal.

The patient data can be diagnosis data according to the International Classification of Diseases, ICD unique medicine codes, demographic data such as age and sex, hospital usage data, number and days of re-hospitalizations, assignment or not of dietitian, as well as results of the Barthel index. The diagnosis returns as a result a value between 0 and 1 of the risk of the patient from suffering oropharyngeal dysphagia, corresponding to that data, being 1 the maximum risk and 0 the minimum.

The system is highly scalable, as it permits implementing at least one query terminal, for example, by clinic/center, and, at least one prediction server, for example, per population 290. Another aspect of the embodiment (the population to the right of the figure) comprises at least one training server 210 which communicates directly with the query terminals 230, without intermediation of an external OD prediction server 220, since the prediction functions are performed also by the training server.

It is understood that the skilled person can configure systems with different topologies. The existence of an application programming interface API which connects the users with the prediction servers, and the use of standards, such as JSON, to transmit and receive information, permits the different users of the system the integration in their own patient management programs, either as a tag, or as a notification when visualizing the clinical record, or any other preferred means. There is complete flexibility on how to integrate the prediction information with the computer systems and compatible with all the existing systems, be it UNIX, Windows or any other.

For example, in one aspect, the communication is direct between the training server 210 and the query terminal 230. This configuration is useful if it is not possible to install an OD server 220 in the population in question. Whilst, in one aspect, the corresponding prediction server 220 responds the queries from the query terminals, after requesting and obtaining the response from the training server 210, in another aspect, it is the prediction server which updates by means of downloading the most recent expert module, and responding the received queries directly. Like this, a server of a computing cluster, can be in charge of training the system continuously. The resulting training models are stored in binary files which are used by the server which performs the predictions. This is connected by means of internal networks, or via internet, and permits clients to perform queries.

In another aspect, a plurality of training servers are implemented, depending on the characteristics of the diagnosis to be performed (whether it relates to OD subclassification, or other type of dysphagia, or even other type of related disease, or whether the volume of data to manage requires it). In one aspect, the training server 210 is centralized and collects data and generates and/or maintains an OD model. In another aspect, the training server 210 is decentralized, with different functions of the expert module and its training distributed in the network.

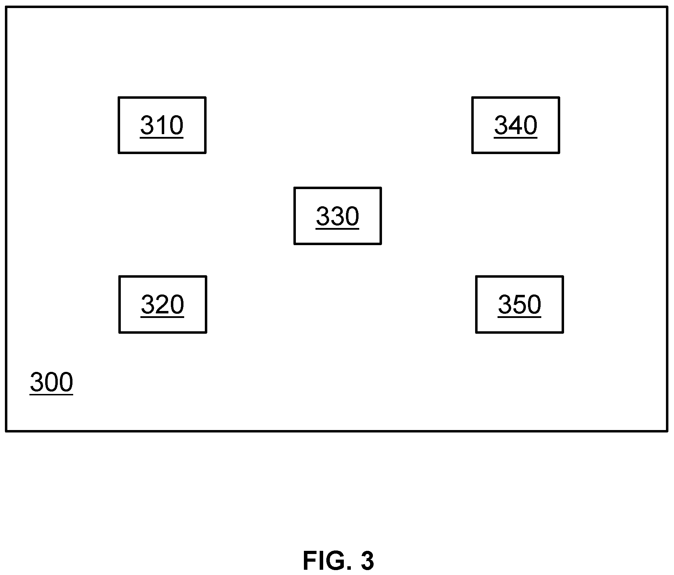

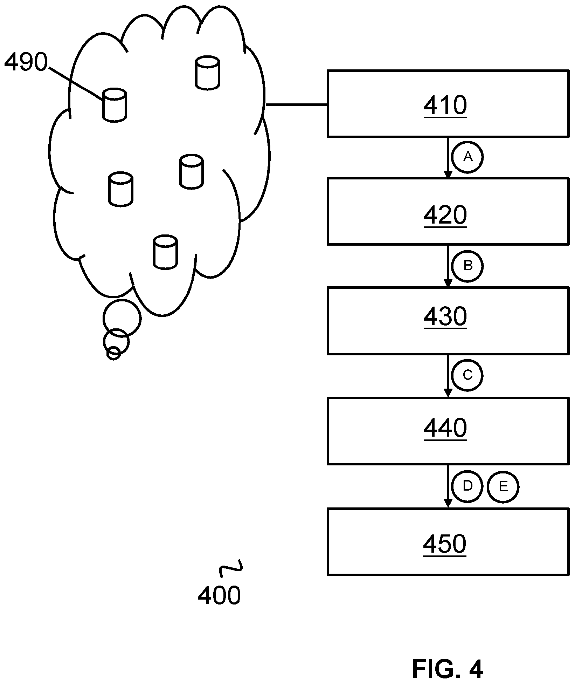

FIG. 3 shows a training server, or training means 300, electronic or digital, according to one aspect of the invention and FIG. 4 shows a method 400 which is performed once the computer program, or program code, is executed, by the at least one training server. The training server executes a training algorithm which comprises five main steps: 1. Database selection 410, 2. Variable selection 420, 3. Expert system training 430, 4. Patient prediction 440, and 5. Training continuation 450. In this aspect, all the functions are performed by the training server. Nevertheless, as discussed with respect to another aspect, it is possible to obtain more scalability and reactivity by responding to the queries by implementing the prediction functions by an independent prediction server.

The first step 410 of database selection is performed by database selection means 310, electronic or digital, configured for, starting from clinical criteria stemming from the acquaintance of the disease, selecting those ICD diagnosis codes (or any other diagnosis codification system) which are closely related to OD, codes of medicines ingested by the user which have been previously linked to OD, demographic variables and finally, clinical variables collected by means of validated instruments. The ICD diagnosis codes (or any other diagnosis codification system) express diagnosis, disease ethology, topography, anatomical pathology and/or nature of the existing lesion by means of codes of between 3-5 digits. The ICD codes were created by the World Health Organization to promote the international comparison of the collected, processing, classification, and presentation of these statistics. ICD is used globally, and in many cases, it is associated, to administrative aspects of the health centers, as well as establish the complexity they assume amongst the different health systems. Since the system uses the international ICD standard codes representative of the International Classification of Diseases, any medical center is capable of issuing a query to the server.

The inventors have realized that a problem exists at the moment of selecting variables due to the extremely large number of variables that can influence OD. Therefore, they have developed the second step 420 of variable selection that is performed by variable selection means 320, electronic or digital, configured for selecting those variables that have a larger OD predictive capacity.

The third step 430 of training is performed by training means 330, electronic or digital, configured for, using the variables selected by the variable selection means, training an expert module by means of automatic learning. That is, the expert module is trained based on automatic learning using the identified variables. In one aspect, each time the training ends, the training result is transferred to the prediction means, either proactively, or periodically, or each time requested by the prediction means.

The fourth step 440 of OD prediction is performed by prediction means 340, electronic or digital, configured for, given a new patient, receive or extract data from its clinical record and execute the prediction based on the data from the record and the models trained by the training server. In one aspect of the embodiment, this step can be configured by the user by means of a risk parameter $\lambda$, by which it can be determined to proceed, or not, to perform a more accurate test.

The fifth step 450 of continuation is performed by the training means 310, electronic or digital, configured to use the collected data of new cases and/or patients which satisfy the established criteria of the screening phase to expand the database and continue training the expert module, with the objective of continuously improving posterior predictions. That is, the new patients are incorporated by repeating the steps of variable selection, training and prediction, gradually improving the accuracy of the algorithm.

In the following each one of the steps of the method are explained in more detail. Going back to the first step 410 of database selection, a training database 490 is obtained starting from the electronic clinical record of the patients which have been hospitalized in one or more hospital institutions. To this effect, relevant medical and statistical criteria have been established in order to exclude from the training database those patients who do not satisfy these criteria:

- Clinical criteria: For example, patients equal to or above 70 years of age.
- Statistical criteria: The collection of data used for training has to be done under statistical criteria. A set of patients have to be selected which, satisfying the clinical criteria, are consecutively admitted in the healthcare center to which the clinical diagnosis method will be systematically applied which will result in both positive as well as negative results. These two points are important, as, in case of selecting patients under other criteria, the system would yield biased results, thereby loosing predictive capacity.

A subset of ICD codes is selected (or any other system of diagnosis codification) from among all possible (>140,000), corresponding to those diseases which, according to the knowledge of the inventors, are related, for example, Parkinson or Alzheimer's disease, cerebral vascular accidents according to location and/or extension, different types of cancer, as well as their location and/or extension, and chronic diseases prevalent in the patient exhibiting OD. This selection can be performed by any skilled person using his OD related knowledge.

Starting from an anonymized database of patients, the following variable selection algorithm is applied, known as Recursive Feature Elimination, to reduce even more the number of codes, resulting in a total between 50 and 150:

- Train the model using all the predictors;
- Determine the model's performance;
- Determine the importance of the variables;
- For each subset of size $S_i$, $i = 1,..., n$, being $n$ the maximum of variables, execute the steps of selecting the most important variables $S_i$, train the model using the training set with the predictors $S_i$, determine the performance of the model;
- Determine the performance profile for each $S_i$;
- Determine the adequate number of predictors;
- Use the model corresponding to the optimum $S_i$, optimum meaning it has better predictive metrics.

The extracted diagnosis codes, as well any other described clinical variables of interest, can be in any format which is exploitable computationally, it being relevant that it is shareable among all users of the system. The International Classification of Diseases has been chosen due to its convenience for diagnosis and the international codes of the medicines. The skilled person can implement a translation to this nomenclature for those cases in which the data exists in some other format. This should enable the exportation of the system to most developed countries around the globe.

Each one of these diagnosis codes and performed tests is introduced into the database, along with its diagnosis date. This time information is used to balance the importance of each diagnosis with the time difference with which it was diagnosed with respect to the prediction moment. During the training process, and according to the training models described, all this information is weighted with its relative interest in order to predict the risk of dysphagia. The larger the quantity of information available, the larger the sensibility, specificity, and predictive value, of the system.

The result of the screening is, for each patient, four groups of variables: (1) demographic variables which define the patient at the instant of its admission (such as, for example, age and sex), (2) undiagnosed clinical variables (such as, for example, hospitalization days in the last month and/or in the last 6 months), (3) results of the Barthel tests, and finally, (4) clinical diagnosis and medicine codes. The Barthel index, BI, is a tool which enables measuring the patient's functionality by means of ten simple questions about the basic activities of the daily routine (eating, walking, hygiene, clothing, and so on). The patient, and/or caregivers, are questioned about each one of the corresponding activities giving a score between 0 and 15 (depending on the activity) with a maximum score of 100 and a minimum of 0. The BI has been standardized globally in the biomedical community as it provides a reliable, fast and simple measurement of the main daily routine activities of the patients. The BI can be performed by any senior healthcare professional.

These variables are stored, and in particular for the second and third variables together with their timestamps, with the objective of incorporating the changes with time that are produced in the patient, for example, the value of the tests, or the presence or absence of the diagnosis, are stored past 3, 6, 12 and 24 months.

To facilitate the understanding of the undergoing processing, X is defined as the global data set, and $S$ the set of variables (1, 2, 3 y 4), being $\#S = n$ the total number of explicative variables included in the data set for a patient $P_j$, $j = 1, ...,m$. Such that $X$ contains for each patient $P_j$, $\#S$ explicative variables, that is, a total of $n \cdot m$ values.

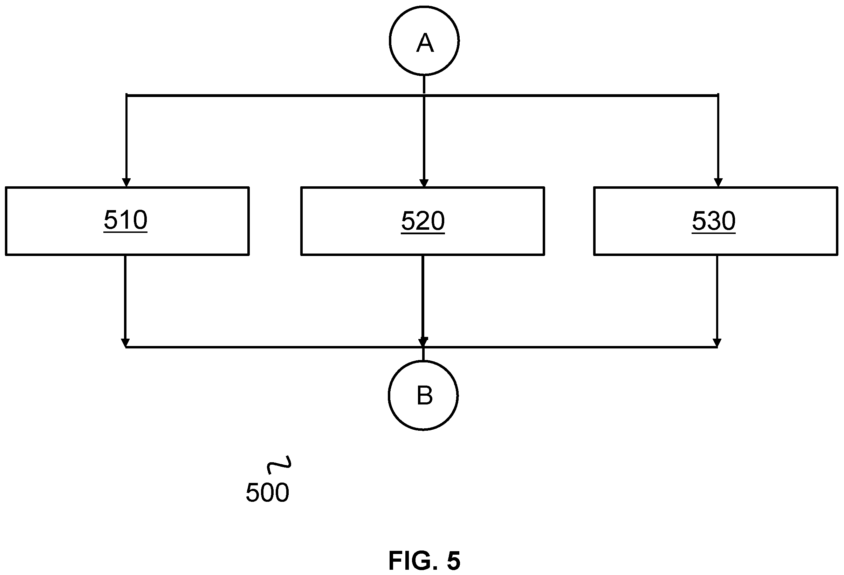

Once all the users who satisfy the criteria are available, as well as all of their clinical data, for each time instant, the explicative variables $\mathcal{S}$ are selected. Once the optimum diagnosis ICD codes are determined, the method proceeds to the next step of selecting 420 the variables with highest predictive capacity. **FIG. 5** shows the process 500 of variable selection, starting from the results (A) of the last step, three models are executed, the first model 510 of random forests, $f_{rf}$, the second model 520 of naive Bayes, $f_{bn}$, and the third model 530, the linear model, $f_{lm}$. In the case of random forests, it is a set of decision trees formed by different resamples obtained by bootstrapping, that is, different training groups are formed using cases that sample randomly and repetitively the global data set. At the end the average result of the prediction of those trees is determined. The decision trees are formed by subdividing the samples in groups of two consecutively. In this manner, after the first partition (or branch), 2 groups exist (or leaves) ..., after the next, 4, and so on successively. The partitions are made by searching, among all of the variables, which division criterion generates two groups with the lowest value according to the Gini index. The naive Bayesian classifier is a particular type of Bayesian network where it is assumed that all the explicative variables are independent amongst one another and dependent on the characteristic that wants to be explained. In the case of the linear model, a classic logistic regression model is constructed. The coefficient estimation is determined by maximizing a likelihood function, assuming that the samples are independent and follow a Bernoulli distribution.

During this variable selection step, the subset $S_k \subseteq S$ is searched for, whose predictive capacity is larger, as identified by a precision index of the generated models. In one aspect, this index identifies the precision by means of the measured area under the curve, Area Under the Receiver Operating Characteristic Curve, AUC, as well as the Matthews' Correlation Coefficient, MCC. Therefore, given the set of predictor variables $\mathcal{S}$, for each $k = 1, ...,p$, the subset of size $k$ is searched which permits constructing the model $f_\ell$: $\ell \in \{lm, rf, bn\}$, with highest predictive capacity, where $lm$, is a linear model, $rf$ is a model based on random forests and $bn$ is a model based on a naive Bayesian network.

Next, the variable selection is performed according to the following method:

 1. For each one of the three training models $f_\ell$:

 1.1. Using the 10-fold Cross Validation training and validation model, 10 data sets $X_1,...,X_{10}$ are created, randomly separating the data set $X$ in 10 parts with approximately the same number of patients in each one. Each one of these sets is used for validation and the remaining 9 for training. This results in 10 validation sets V = {$V_1$, ..., $V_{10}$} and 10 training sets T = {$T_1$, ..., $T_{10}$}. Therefore, for example, $X_1 = V_1 \cup T_1$;
 1.2. For each one of the 10 sets $X_i$:

 1.2.1. A model is trained with all of the variables in $S$ using the elements of $T_i$;
 1.2.2. The variables are ordered by predictive relevance;
 1.2.3. For each $k$:

1.2.3.1. A model is generated using the first $k$ variables with highest predictive capacity.
1.2.3.2. The model is validated using the elements of $V_i$;
1.2.3.3. The variables are reordered by importance as a function of the new model.

2. For each training model $f_\theta$ it is verified whether $k$ has obtained better results (taking the mean of each 10 repetitions), and the optimum variables are determined of each subset.

As a result, the set of variables are obtained which has the highest explicative capacity according to the lineal model

$$\mathcal{S}_{lm} \subseteq \mathcal{S},$$

the random forests model

$$\mathcal{S}_{rf} \subseteq \mathcal{S},$$

and the naive Bayesian classifier model

$$\mathcal{S}_{bn} \subseteq \mathcal{S},$$

where, logically,

$$\#\mathcal{S}_{lm}, \#\mathcal{S}_{rf}, \#\mathcal{S}_{bn} \leq \#\mathcal{S}.$$

Finally, the variables are selected which are included in the subset and have demonstrated best predictive capabilities, in addition to the intersection of the other two. That is:

$$\hat{\mathcal{S}} = \mathcal{S}_1 \cup (\mathcal{S}_2 \cap \mathcal{S}_3) \qquad\qquad \text{[equation 1]}$$

where $\hat{\mathcal{S}}$ is the subset that is used in the following steps of the algorithm, and the expected output of the described algorithm, and $\mathcal{S}_i$, $\forall i \in \{1, 2, 3\}$ is the subset of variables obtained in the previous steps, being $\mathcal{S}_1$ the one which has obtained the best precision metrics, and $\mathcal{S}_3$ the one which has obtained the worst metrics. In one aspect, in which the best model is the linear model, all of the variables of the linear model which have produced the best results are included in addition to those common with the other two models, naive Bayesian and random forests.

The use of the linear model has demonstrated to be highly effective as a variable selector, however, it only identifies the linear relationships, which is solved by including the random forests and Bayesian network models, which are not based on the linear relationships between the variables but one their joint distribution, as occurs with dysphagia, where the relationship between having dysphagia or not and the majority of the used variables is not linear. Both random forests as well as Bayesian networks have complementary suppositions which permit that using both to capture the majority of existing relationships. Finally, that model which has the best predictive capacity is the one which best captures the relationships among the variables, that is the reason why all of them are included. The other two models also capture relationships, but only those present in both are included in order not to overcomplicate the system, which would later translate in performance loss.

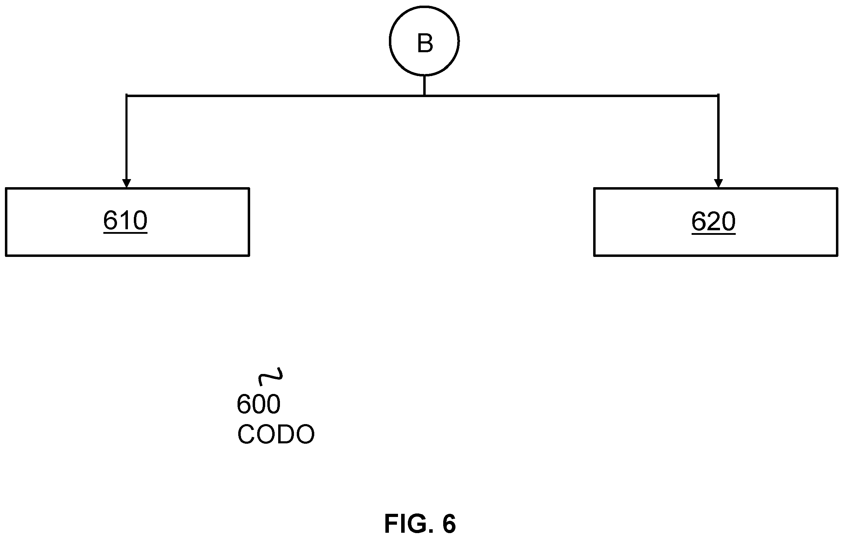

Once the best variables have been selected (B), these are used in the next step to train 430 the expert module. **FIG. 6** shows the training step of the expert module according to one aspect of the invention, within the global process named herein as Optimum Oropharyngeal Dysphagia Screening, CODO, wherein a first model 610 of random forests and a second model 620 of Bayesian networks are trained. Both models are managed and maintained by their updating using updated data of the same patients or data of new patients. The Bayesian network second model can be, in one aspect, a regular Bayesian model or, in another aspect, a naive Bayesian model, or other type of Bayesian network.

The set of variables $\hat{S}$ permits proceeding with the training of the expert models. A random forests model is generated 610 in the first step and next, in a second step, a Bayesian network model is generated 620 (regular, naive, or other), in both cases using the set of patients X and their explicative variables $\hat{S}$, however, assuming that not all the variables have to be necessarily independent amongst one another.

Based on these generated and trained models, the risk of OD of any patient with an electronic clinical record, eCR, can be established. The implementation of eCR enables storing and processing all the clinical information of the patient (diagnosis, clinical data, professional annotations, diagnosis tests). This digitalization process enables any senior healthcare professional to consult such information independently from geographic location, and subsequent usage of this information by the system object of this invention.

Once the expert module has been trained, it is used in the screening phase to predict the risk of suffering from OD. This fourth prediction step 440 starts by receiving (C), by the prediction server, a query related to a patient, or group of patients. Using the patient's data as input, the expert module determines the risk parameter of suffering from OD for this patient in question, returning it as output.

The probability of suffering dysphagia P(d = *yes)* is determined as a function of a random forests first model and a Bayesian network second model. The computation is different for the random forests than for the Bayesian networks. In the random forests, the estimation is performed according to the individual votes of each tree that conforms the forests. That is:

$$P(d = yes) = \frac{B_{yes}}{B_{yes}+B_{no}} \qquad \text{[equation 2]}$$

where $B_{yes}$ and $B_{no}$ are the number of trees that predict dysphagia yes and no respectively.

In the case of Bayesian networks, the probability of suffering from dysphagia can be computed based on the conditional probability of the roots (those variables which point towards dysphagia), that is:

$$P(d = yes) = \prod_{\mathfrak{P}_i \in \mathfrak{P}} P(d = yes | \mathfrak{P}_i = \mathfrak{p}_j) \qquad \text{[equation 3]}$$

where

$$\mathfrak{P}_i \in \mathfrak{P}$$

are the roots of the dysphagia variable and $\mathfrak{p}_j$ are the values they adopt. Note that $\mathfrak{P}$ depends of the graph, and that, how $\mathfrak{P}$ is estimated depends on the training algorithm.

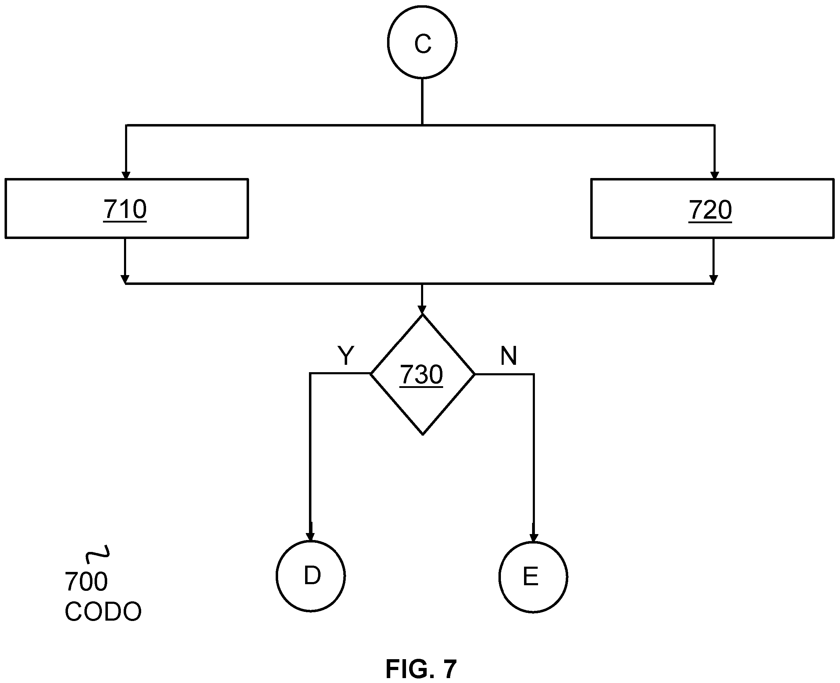

FIG. 7 shows the last prediction step 700 according to one aspect of the invention, within the global process named herein as Optimum Oropharyngeal Dysphagia Screening, CODO, which determines the risk of suffering OD of each hospitalized patient. For the prediction of a patient, the set of variables *S* is extracted from the patient's clinical record, and it is evaluated by the expert module. If both systems coincide in the prediction, that risk value is returned (D) (dysphagia Yes/No). On the other hand, if they are different, then the risk parameter λ is used (E) to decide if it is convenient to perform the test on the patient. If the risk is high, that is, above 50%, then it is recommended to perform the second 120, and possibly third 130, diagnosis phases of FIG. 1 or FIG. 8, on the patient, which are tests with higher sensibility and specificity. Otherwise, the rest of the diagnosis process is aborted (E).

The OD risk prediction step starts by the prediction means receiving a query (C) from a query terminal. As described, the prediction means are part of the training server in one aspect, or exist separately and independently in a prediction server, in another aspect. Periodically, the prediction means receive the latest updated model from the training means. On one hand, the risk of OD is determined 710 according to a random forests first model and the risk of OD is determined 720 according to a Bayesian network second model, in a similar manner as effected whilst training the model according to FIG. 5. The Bayesian network second model can be, in one aspect, a regular Bayesian model, whilst in another aspect, it can be a naive Bayesian model, or other type of Bayesian network. Next, it is determined 730 whether the results of both is the same, in the sense that if in both cases it has been determined that the risk of suffering from OD is above 50%. In case positive, it is determined (D) that the patient suffers from OD with enough probability to continue with the OD diagnosis process.

Otherwise, if the evaluation performed by the random forests model is different from that of the Bayesian network model, for example, if either of them results in a risk which is equal to or lower than 50%, then a risk parameter $\lambda$ is applied (E) enabling the definition of an acceptable risk level associated to detecting, or not, the OD, and the execution of both models is repeated. It is determined that there is positive risk of suffering from oropharyngeal dysphagia if the result of both models is above 50%, or it is determined that there is no risk of suffering from oropharyngeal dysphagia if the result of both models is equal to or below 50%. This innovative process is named herein as Optimum Oropharyngeal Dysphagia Screening, CODO.

The level of the risk parameter $\lambda$ defines an inverse relation between false negatives and false positives. Thereby increasing or reducing the population of patients with OD risk depending on the level set for this parameter, there existing a larger percentage of patients in the smaller populations, and smaller in the larger populations. In other words, the decision-making process is forced by assigning a higher weighting to one model over the other.

The parameter $\lambda \in < [0,1]$, called the risk parameter, serves to establish the risk associated to not detecting the dysphagia. This value enables changing the ratio between false positives and false negatives at the time of diagnosis. If a high value is selected for the risk parameter, for example, $\lambda > 0.7$, the level of false negatives decreases in detriment of the false positives. On the other hand, if the risk assumed is low, for example, $\lambda < 0.4$, the index of false positives increases in detriment of the false negatives. In other words, in the first case, less people are classified as having a risk of dysphagia, but among them, the percentage of suffering from it will be larger than in the second case, where more people are classified as risky, but among them, the percentage will be lower. It has to be taken into account that the detrimental effect associated with not recognizing a patient as really being affected by OD (false negative) is far larger that the detrimental effects of a false positive, who will undergo an instrumental test.

This parameter enables modulating the screening precision required according to circumstances. In case a high probability of positive diagnosis tests is necessary, in that case, by using $\lambda$, the user can rank the patients in order of risk, and perform the tests only on those with the highest probability of suffering from dysphagia. On the other hand, the system users can independently define their $\lambda$ of preference, which permits each user to establish, according to interests and policy, different risk levels according to their health policies. One of the best aspects of this approach is that an interval $\mathcal{J}$ exists for which the precision metrics are invariant, and the sum of false positives and false negatives is maintained constant. This implies that whilst $\lambda$ exists in $\mathcal{J}$, no predictive capacity is lost, which gives the users a lot of capacity to decide how they want to manage the risk. It should be noted that $\mathcal{J}$ is not unknown, and it is readily estimated during the training step.

Therefore, the different aspects of the described algorithm CODO enables improving the precision whilst diagnosing OD, to perform an automatized method reducing the required human resources in the first screening step, optimize the first screening step in OD diagnosis to consume important resources only in those patients who have high probability of suffering the condition, according to the characteristics of the population undergoing the diagnosis, dynamically vary the volume of the population to analyze according to pre-established objectives and resource restrictions.

CODO is a considerable improvement over classic algorithms, which never surpass a final screening precision of 60% - 65 %. On the other hand, by implementing the CODO algorithm according to the invention, the precision increases to 68% - 69%. In other words, globally a higher screening precision is obtained.

On the other hand, the inventors have also realized the excessive consumption in computational resources (for example, in terms of computations per minute, global time until reaching a solution, the consumed energy until reaching a solution) which this combination of subroutines of the screening algorithm involves which results in a higher precision. The most visible parameter for the user is the total time until reaching to the solution of the screening method.

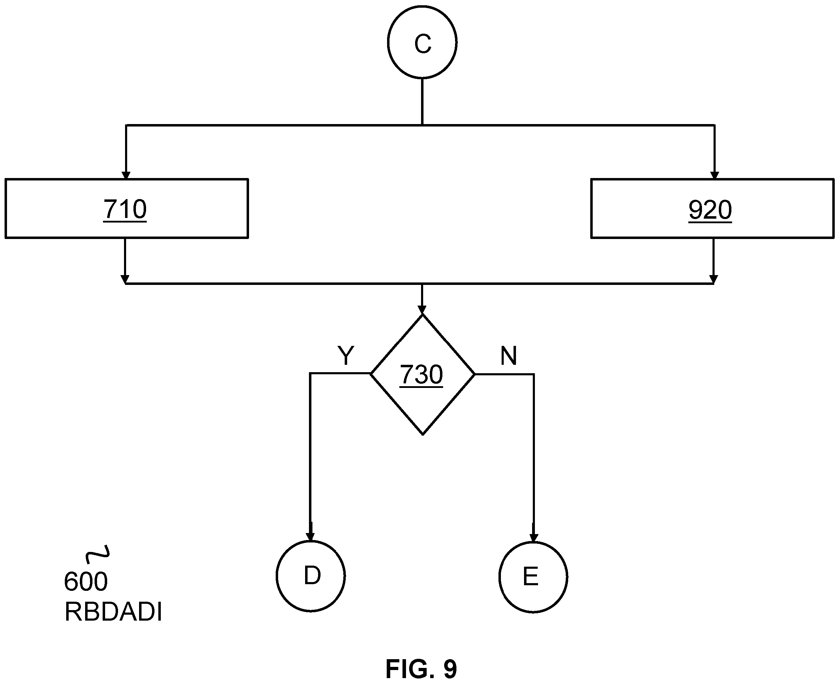

With the objective of reducing the consumption of computational resources, the CODO screening algorithm has been modified replacing the Bayesian network model with an innovative development called herein the High Information Density Disperse Bayesian Network, RBDADI. In particular, this is represented in step 620 of FIG. 6 or in step 720 of FIG. 7, in which steps the Bayesian network model has been replaced by the RBDADI model described in the following. **FIG. 9** shows the last prediction step 900 according to one aspect of the invention in which establishing the risk of OD of each hospitalized patient comprises the RBDADI model 920.

The identified problem of the conventional Bayesian networks is that, given a Bayesian network, formed by the directed acyclic graph $\mathcal{G} < V, E >$ where $V \in \{V_1, ..., V_n\}$ are the vertices or variables and $E \in \{E_1, ..., E_m\}$ are the arcs which determine the relations that exist amongst them as well as their direction, if the elements forming E are unknown, it is possible to approximate them given a data set $D$, $D \in \mathbb{R}^{n \times o}$, where $o$ is the number of observations available. A very common manner of estimating the vertices that conform V is using a greedy search algorithm which maximizes

the likelihood function $\mathcal{L}(\mathcal{G}/$ D), adding and removing vertices.

Given the graph $\mathcal{G}$ with an empty set E and a data set D, the conventional Bayesian network implements a known greedy search algorithm:

1. Whilst $\mathcal{L}' \geq \mathcal{L}$ do:

    a. Calculate $\mathcal{L}(\mathcal{G}/D)$

    b. For each possible $E_p$, $p$ = 1, ..., $l$, of vertices pairs $\{V_i, V_j\} \in V$, for $\mathcal{G}' = \mathcal{G} \cup E_p$ and for $\mathcal{G}' = \mathcal{G} \backslash E_p$

    calculate $\mathcal{L} = \mathcal{L}(\mathcal{G}'/D)$

    c. Select $E_p$ whose $\mathcal{L}(\mathcal{G}'/D)$ is largest and update $\mathcal{G}$, $\mathcal{G} = \mathcal{G}'$. Calculate $\mathcal{L}' = \mathcal{L}(\mathcal{G}/D)$

2. Store $\mathcal{G}$ as $\mathcal{G}^*$

3. Randomly select $E_i$ and add them or remove them randomly from $\mathcal{G}$

4. Repeat step 1.

5. Whilst $\mathcal{L}(\mathcal{G}^*/D) <= \mathcal{L}(\mathcal{G}/D)$:

    a. Repeat steps 2, 3 and 4

6. Return $\mathcal{G}$.

This algorithm searches one by one looking for the option which increases the likelihood of $\mathcal{G}$. That is, how probable it is to observe D given $\mathcal{G}$. Since this algorithm can result in a local maximum, the network is perturbed in steps 2, 3, and 4 to start anew, until it is not possible to find a better maximum, resulting in the decisive graph. On the other hand, since in the OD screening process potentially a lot of variables are used, the Bayesian networks are very connected (high information density) and therefore produce a worse performance in the prediction tasks. Also, given their larger complexity, the time necessary to perform the computations is increased since the resulting graph contains a high density of vertices and information, reflecting in a large consumption of computational resources to process them and converge to a final solution.

With the objective of providing a more efficient search algorithm, whose use for predicting consumes less computational resources, a modification of said greedy search algorithm is proposed. The suggested modification supposes limiting the vertices that can be incorporated according to two criteria, the shared information between both variables and the predictive capacity of the network.

Given a graph $\mathcal{G}$ with an empty set E and a data set D, the following RBDADI algorithm is implemented:

1. Divide randomly D in exclusive subsets $D_t$ and $D_v$
2. Whilst

$$\mathcal{M} \leq \mathcal{M}',$$

do:

    a. Use $D_t$ in algorithm 1 to find $\mathcal{G}$

    b. Validate $\mathcal{G}$, calculating Mathews' correlation coefficient (MCC) given $D_v$, that is,

$$\mathcal{M} = MCC(\mathcal{G}/D_v)$$

for the variable of interest (dysphagia in our case).

c. Given $\mathcal{G}$ , for each $E_i \in E$, calculate $H_i$. Where $H_i$. Is Shannon's mutual information index.

d. Include $E_i$ with the minimum value of $H_i$ in *B*.

e. Repeat algorithm 1 prohibiting the vertices included in B and obtain $\mathcal{G}'$.

f. Calculate $\mathcal{M}'$,

$$\mathcal{M}' = MCC(\mathcal{G}'/D_v)$$

g. Update $\mathcal{G}$ , $\mathcal{G} = \mathcal{G}'$ .

3. Return $\mathcal{G}$ .

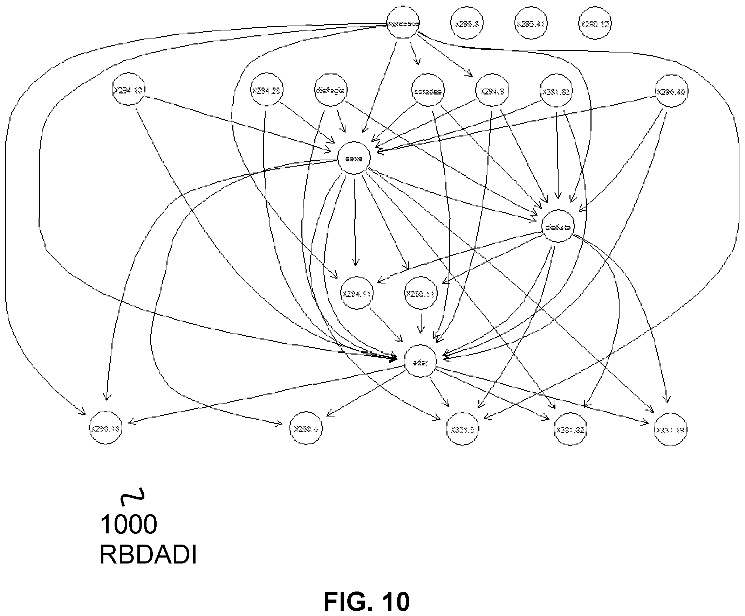

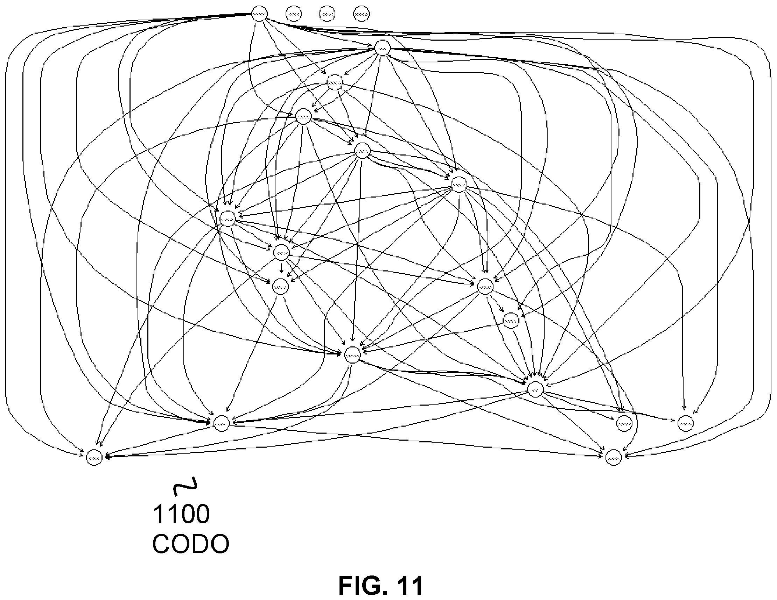

As a result, in this algorithm those connections which contribute the least relevant information are excluded, according to Shannon's mutual information index. Therefore, the remaining vertices join variables which share larger information, thereby favoring, in general, the existence of less roots for each variable, thereby resulting in less need for the computation of equation 3, that is, since the cardinal of $\mathfrak{P}$ is lower, less multiplications are performed. In other words, the prediction determination is a function of Shannon's mutual information index. This improvement can be observed in FIGs. 10 and 11. **FIG. 11** is a representation of the number of existing arcs in the CODO network, whilst **FIG. 10** is a representation in the number of arcs existing in the RBDADI network. As can be seen, there are a smaller number of arcs in the RBDADI network than in the CODO network, however the information contained in the network is similar, or equivalently, the information density in terms of arcs is higher, resulting in less computational resources being necessary to obtain the same screening precision.

This improvement can be observed correspondingly in the following comparative table of experimental results. **TABLE 1** shows, based on the same input parameters, the result in consumption of computational resources, in this case in terms of execution time until reaching the solution, using the CODO algorithm of the invention in comparison to the RBDADI algorithm of the invention:

TABLE 1 - Comparison of computational resources

| № variables | Time CODO | Time RBDADI | Difference Time | Precision CODO | Precision RBDADI | Density CODO | Density RBDADI |
|---|---|---|---|---|---|---|---|
| 5 | 0.769 ± 0.363 | 0.459 ± 0.168 | 40.323% | 0.694 ± 0.011 | 0.675 ± 0.011 | 15 ± 0 | 11 ± 0 |
| 10 | 0.719 ± 0.121 | 0.587 ± 0.272 | 18.448% | 0.686 ± 0.016 | 0.687 ± 0.008 | 48 ± 0 | 25 ± 2 |
| 20 | 1.054 ± 0.132 | 0.757 ± 0.238 | 28.235% | 0.677 ± 0.017 | 0.691 ± 0.012 | 108 ± 14 | 47 ± 5 |
| 50 | 12.068 ± 16.54 | 4.267 ± 3.43 | 64.645% | 0.686 ± 0.016 | 0.684 ± 0.012 | 202 ± 21 | 129 ± 11 |

The time is represented in milliseconds per processor nucleus per query. The tests have been repeated ten times with an Intel® Core™ i7-6700HQ @ 2.60GHz processor with 4 physical nuclei and 8 virtual ones. The programming language used to perform the experiments has been R, containing 5159 hospitalized patient records using the original database. The experiments have been performed randomly selecting 80% of the cases to train the network and 20% to calculate these results. In this case the CODO network has been implemented using as a second model a Bayesian network trained with the *greedy search Hill climbing* algorithm (called the regular Bayesian network, in this description).

As can be observed, with practically the same precision results, the time necessary to perform the computations is approximately 37.913% on average lower for the RBDADI model when compared to the CODO model. Inversely, the computation speed is correspondingly higher for the RBDADI model in comparison to the CODO model. Also, the more variables are taken into account, the larger is the improvement in time reduction or speed increase. It can be also observed how the network density in the RBDADI network is on average 43% lower that the density of the CODO network. Therefore, the RBDADI model represents a substantial improvement in terms of reduction in the consumption of computational resources, or reduction in computational time, or increase in computation speed, whilst performing the OD screening. These parameters are measurements which represent the technical improvement of the digital system whilst performing complex computations with an immense quantity of data.

In one aspect, the centralized training server encompasses the improvements in the expert system (reducing the time necessary for the continuous training), whilst the plurality of prediction servers serve local populations according to geographic zones (reducing the time necessary for the prediction), permitting the efficient geographical escalation of the system, the sensible data are stored in a unique centralized training server, all of this enabling the early detection of a patient suffering from OD, improving its possibilities of aid and treatment, and reducing the associated detrimental effects of the treatment. 1. A cluster or server with a high computation capacity focused only in training the system which serves the training results to the rest of the prediction servers, enabling minimizing the time necessary for training and minimizing the time necessary for performing prediction for the user. 2. Separate the training server from the prediction server enabling having diverse prediction servers located in geographical zones close to the users. For example, there can be one or more prediction servers in Japan giving coverage to Asia, one or many in the United States giving coverage to North America and one or many in Europe giving coverage to the European continent, the system thereby being scalable and permitting adapting very rapidly to user demand. 3. The separation between the training and the prediction servers improves security and adds security to the sensitive data, which would be held only in the training server.

There are several additional benefits of the system. An automatic screening which considerably improves the number of detected patients at an early stage of the disease, when it is the most important for the health of the patient and represents important benefits for the public health system. The early detection of dysphagia reduces the number of patient hospitalizations, as well as the number of pneumonias that can be acquired, thus considerably reducing the detrimental administrative effects derived from such treatments.

Furthermore, it is to be understood that the embodiments, realizations, and aspects described herein may be implemented by various means in hardware, software, firmware, middleware, microcode, or any combination thereof. Various aspects or features described herein may be implemented, on one hand, as a method or process or function, and on the other hand as an apparatus, a device, a system, or computer program accessible from any computer-readable device, carrier, or media. The methods or algorithms described may be embodied directly in hardware, in a software module executed by a processor, or a combination of the two.

The various means may comprise software modules residing in RAM memory, flash memory, ROM memory, EPROM memory, EEPROM memory, registers, hard disk, a removable disk, a CD-ROM, or any other form of storage medium known in the art.

The various means may comprise logical blocks, modules, and circuits may be implemented or performed with a general purpose processor, a digital signal processor (DSP), and application specific integrated circuit (ASIC), a field programmable gate array (FPGA), or other programmable logic device, discrete gate or transistor logic, discrete hardware components, or any combination thereof designed to perform the functions described. A general-purpose processor may be a microprocessor, but in the alternative, the processor may be any conventional processor, controller, microcontroller, or state machine.

The various means may comprise computer-readable media including, but not limited to, magnetic storage devices (for example, hard disk, floppy disk, magnetic strips, etc.), optical disks (for example, compact disk (CD), digital versatile disk (DVD), etc.), smart cards, and flash memory devices (for example, EPROM, card, stick, key drive, etc.). Additionally, various storage media described herein can represent one or more devices and/or other machine-readable media for storing information. The term "machine-readable medium" can include, without being limited to, various media capable of storing, containing, and/or carrying instruction(s) and/or data. Additionally, a computer program product may include a computer readable medium having one or more instructions or codes operable to cause a computer to perform the functions described herein.

What has been described above includes examples of one or more embodiments. It is, of course, not possible to describe every conceivable combination, or permutation, of components and/or methodologies for purposes of describing the aforementioned embodiments. However, one of ordinary skill in the art will recognize that many further combinations and permutations of various embodiments are possible within the general inventive concept derivable from a direct and objective reading of the present disclosure. Accordingly, it is intended to embrace all such alterations, modifications and variations that fall within scope of the appended claims.

Also, the skilled person understands that the different embodiments can be implemented in hardware, software, firmware, middleware, microcode, or any other combination of the same. Various of the described aspects or characteristics can be implemented, on one hand, as a process or method or function, and on the other hand, as an apparatus, device, system, or computer program accessible by any device readable by a computer, carrier or means. The methods and algorithms described can be implemented directly in hardware, in a software module executed by a processor, or a combination of both. The various means can comprise software modules resident in RAM memory, flash memory, ROM memory, EPROM memory, EEPROM registries, hard disk, removable disk, a CD-ROM, or any other type storage means known in the art.

The various means can comprise logical blocks, modules, and circuits can be implemented or performed by a general purpose processor, a digital signal processor (DSP), an application specific integrated array (ASIC), a field programmable gate array (FPGA), or other programmable logic device, discrete gate or transistor logic, discrete hardware components, or any combination thereof designed to perform the functions described. A general-purpose processor may be a microprocessor, but in the alternative, the processor may be any conventional processor, controller, microcontroller, or state machine or embedded processor.

The various means may comprise computer-readable media including, but not limited to, magnetic storage devices (for example, hard disk, floppy disk, magnetic strips, etc.), optical disks (for example, compact disk (CD), digital versatile disk (DVD), etc.), smart cards, and flash memory devices (for example, EPROM, card, stick, key drive, etc.). Additionally, various storage media described herein can represent one or more devices and/or other machine-readable media for storing information. The term "machine-readable medium" can include, without being limited to, various media capable of storing, containing, and/or carrying instruction(s) and/or data. Additionally, a computer program product may include a computer readable medium having one or more instructions or codes operable to cause a computer to perform the functions described herein.

What has been described above includes examples of one or more embodiments. It is, of course, not possible to describe every conceivable combination, or permutation, of components and/or methodologies for purposes of describing the aforementioned embodiments. However, one of ordinary skill in the art will recognize that many further combinations and permutations of various embodiments are possible within the general inventive concept derivable from a direct and objective reading of the present disclosure. Accordingly, the main embodiments have been described, under the understanding that they comprise all other combinations, variations and modifications.

In the following, certain additional aspects or examples are described:
A digital system for the universal, systematic and optimized screening of oropharyngeal dysphagia, which comprises at least one training server, at least one prediction server, and at least one query terminal configured to request the determination of the risk of suffering from oropharyngeal dysphagia by at least one patient, wherein the request comprises data of the at least one patient; wherein the at least one training server comprises: at least some digital means of selecting databases configured for selecting ICD codes related to oropharyngeal dysphagia; at least some digital means of selecting variables configured for selecting the variables with a higher capacity of predicting oropharyngeal dysphagia as a function of the selected ICD codes; and at least some digital means of training configured for training an expert module as a function of the selected variables; wherein the at least one prediction server comprises at least some digital prediction means configured for determining the risk of suffering from oropharyngeal dysphagia of at least one patient using the received data of the at least one patient as input to the expert module, wherein the risk of suffering from oropharyngeal dysphagia is determined as a function of a random forests first model and a Bayesian network second model.

The system, wherein the system does not comprise the at least one prediction server, and the at least one training server comprises additionally a training server comprising additionally the at least some digital prediction means. The system, wherein the data of the at least one patient comprises its electronic clinical record. The system, wherein the at least one training server is a centralized server or a distributed server. The system, wherein the digital means for database selection are configured for selecting a subset of ICD codes, and filter them by applying a recursive feature elimination algorithm. The system, wherein the digital means for database selection are configured for tagging the selected codes with a time stamp. The system, wherein the digital means for variable selection are configured for, using the selected ICD codes as input parameters, executing a random forests first model, a naive Bayesian second model, and a third linear model, and determining all the variables of the best model additionally to those that are jointly in both of the other two models, for selecting the variables with the largest predictive capacity. The system, wherein the digital training means are configured for executing an optimized oropharyngeal dysphagia screening process, CODO, according to a random forests first model, and a Bayesian network second model, for training the expert module. The system, wherein the digital prediction means are configured for executing an optimized oropharyngeal dysphagia screening process, CODO, comprising downloading the most recent expert module update and using the data of the at least one patient as input to the random forests first model of the expert module and using the data of the at least one patient as input to the Bayesian network second model of the expert module, and determining that there is risk of suffering from oropharyngeal dysphagia if the result of both models is above 50%. The system, wherein the digital training means are configured for executing a random forests first model, and a high information density disperse Bayesian network second model, RBDADI, which comprises Shannon's mutual information index, for training the expert module. The system, wherein the digital prediction means are configured for downloading the latest update of the expert module and using the data of the at least one patient as input to the random forests first model of the expert module and using the data of the at least one patient as input to the high information density disperse Bayesian network second model, RBDADI, as a function of Shannon's mutual information index, of the expert module, and determining that there is positive risk of suffering from oropharyngeal dysphagia if the result of both models is above 50%. The system, wherein, if the result of either model does not exceed 50%, the prediction means are configured for applying a risk parameter $\lambda$, between 0 and 1, and repeat the execution of both models and determining that there is positive risk of suffering from oropharyngeal dysphagia if the result of both models is above 50%, or determining that there is no risk of suffering from oropharyngeal dysphagia if the result of both models is equal to or below 50%.

A method of optimized screening of oropharyngeal dysphagia in a digital system that comprises at least one training server, at least one prediction server, and at least one query terminal, comprising the method: requesting, by the query terminal, the determination of the risk of suffering from oropharyngeal dysphagia by at least one patient, wherein the request comprises data of the at least one patient; selecting, by at least some digital means for database selection, some ICD codes related to oropharyngeal dysphagia; selecting, by at least some digital means for variable selection, the variables that have a largest capacity of predicting oropharyngeal dysphagia as a function of the selected ICD codes; training, by at least some digital training means, an expert module as a function of the selected variables; and determining, by at least some digital prediction means, the risk of suffering from oropharyngeal dysphagia of the at least one patient using the data received of the at least one patient as input to the expert module, wherein the risk of suffering from oropharyngeal dysphagia is determined as a function of a random forests first model and a Bayesian network second model.

The method, wherein the at least one training server comprises the at least some digital prediction means, or wherein the at least some digital prediction means are configured externally, in at least one prediction server. The method, wherein the data of the at least one patient comprises its clinical record. The method, wherein the training of the expert module is performed in a centralized or distributed manner. The method, wherein the database selection comprises selecting a subset of ICD codes, and filtering them by applying a recursive feature elimination algorithm. The method, wherein the database selection comprises tagging the selected codes with a time stamp. The method, wherein the variable selection comprises, using the selected codes as input parameters, executing a random forests first model, a naive Bayesian second model, and a linear third model, and determining all the variables of the best model additionally to those that are jointly in the other two models, for selecting the variables with highest predictive capacity. The method, wherein the training comprises executing an optimized oropharyngeal dysphagia screening process, CODO, as a function of a random forests first model, and a Bayesian network second model, for training the expert module. The method, wherein the prediction comprises executing an optimized oropharyngeal dysphagia screening process, CODO, comprising downloading the latest update of the expert module and using the data of the at least one patient as input to the random forests first model of the expert module and using the data of the at least one patient as input to a Bayesian network second model of the expert module, and determining that there is positive risk of suffering from oropharyngeal dysphagia if the result of both models is above 50%. The method, wherein the training comprises executing a random forests first model, and a high information density disperse Bayesian network second model, RBDADI, which comprises Shannon's mutual information index, for training the expert module. The method, wherein the prediction comprises downloading the latest expert module update and using the data of the at least one patient as input to the random forests first model of the expert module and using the data of the at least one patient as input to the high information density disperse Bayesian network second model, RBDADI, as a function of Shannon's mutual information index, of the expert module, and determining that there is positive risk of suffering from oropharyngeal dysphagia if the result of both models is above 50%. The method, wherein, if the result of either of both models does not exceed 50%, applying a risk parameter $\lambda$, between 0 and 1, and repeating executing both models and determining that there is positive risk of suffering from oropharyngeal dysphagia if the result of both models is above 50%, or determining that there is no risk of suffering from oropharyngeal dysphagia if the result of both models is equal to or below 50%.

A computer program, which comprising instructions which, once executed on a processor, performs the method steps.

Computer readable means comprising instructions which, once executed on a processor, performs the method steps.

**Claims**

1. A digital system for the universal, systematic and optimized screening of oropharyngeal dysphagia, which comprises at least one training server, at least one prediction server, and at least one query terminal configured to request the determination of the risk of suffering from oropharyngeal dysphagia by at least one patient, wherein the request comprises data of the at least one patient;

   wherein the at least one training server comprises:

   at least some digital means of selecting databases configured for selecting ICD codes related to oropharyngeal dysphagia;
   at least some digital means of selecting variables configured for selecting the variables with a higher capacity of predicting oropharyngeal dysphagia as a function of the selected ICD codes; and
   at least some digital means of training configured for training an expert module as a function of the selected variables;

wherein the at least one prediction server comprises at least some digital prediction means configured for determining the risk of suffering from oropharyngeal dysphagia of at least one patient using the received data of the at least one patient as input to the expert module, wherein the risk of suffering from oropharyngeal dysphagia is determined as a function of a random forests first model and a Bayesian network second model.

2. The system of claim 1, wherein the system does not comprise the at least one prediction server, and the at least one training server comprises additionally a training server comprising additionally the at least some digital prediction means.

3. The system of claim 2, wherein the data of the at least one patient comprises data from its clinical record.

4. The system of claim 3, wherein the at least one training server is a centralized server or a distributed server.

5. The system of claim 4, wherein the digital means for database selection are configured for selecting a subset of ICD codes, and filter them by applying a recursive feature elimination algorithm.

6. The system of claim 5, wherein the digital means for database selection are configured for tagging the selected codes with a time stamp.

7. The system of claim 4, wherein the digital means for variable selection are configured for, using the selected ICD codes as input parameters, executing a random forests first model, a naive Bayesian second model, and a third linear model, and determining all the variables of the best model additionally to those that are jointly in both of the other two models, for selecting the variables with the largest predictive capacity.

8. The system of claim 4, wherein the digital training means are configured for executing an optimized oropharyngeal dysphagia screening process, CODO, according to a random forests first model, and a Bayesian network second model, for training the expert module.

9. The system of claim 4, wherein the digital prediction means are configured for executing an optimized oropharyngeal dysphagia screening process, CODO, comprising downloading the most recent expert module update and using the data of the at least one patient as input to the random forests first model of the expert module and using the data of the at least one patient as input to the Bayesian network second model of the expert module, and determining that there is risk of suffering from oropharyngeal dysphagia if the result of both models is above 50%.

10. The system of claim 4, wherein the digital training means are configured for executing a random forests first model, and a high information density disperse Bayesian network second model, RBDADI, which comprises Shannon's mutual information index, for training the expert module.

11. The system of claim 4, wherein the digital prediction means are configured for downloading the latest update of the expert module and using the data of the at least one patient as input to the random forests first model of the expert module and using the data of the at least one patient as input to the high information density disperse Bayesian network second model, RBDADI, as a function of Shannon's mutual information index, of the expert module, and determining that there is positive risk of suffering from oropharyngeal dysphagia if the result of both models is above 50%.

12. The system of claim 9 or 11, wherein, if the result of either model does not exceed 50%, the prediction means are configured for applying a risk parameter $\lambda$, between 0 and 1, and repeat the execution of both models and determining that there is positive risk of suffering from oropharyngeal dysphagia if the result of both models is above 50%, or determining that there is no risk of suffering from oropharyngeal dysphagia if the result of both models is equal to or below 50%.

13. A method of optimized screening of oropharyngeal dysphagia in a digital system that comprises at least one training server, at least one prediction server, and at least one query terminal, comprising the method:

requesting, by the query terminal, the determination of the risk of suffering from oropharyngeal dysphagia by at least one patient, wherein the request comprises data of the at least one patient;

selecting, by at least some digital means for database selection, some ICD codes related to oropharyngeal dysphagia;

selecting, by at least some digital means for variable selection, the variables that have a largest capacity of predicting oropharyngeal dysphagia as a function of the selected ICD codes;

training, by at least some digital training means, an expert module as a function of the selected variables; and determining, by at least some digital prediction means, the risk of suffering from oropharyngeal dysphagia of the at least one patient using the data received of the at least one patient as input to the expert module, wherein the risk of suffering from oropharyngeal dysphagia is determined as a function of a random forests first model and a Bayesian network second model.

14. The method of claim 13, wherein the at least one training server comprises the at least some digital prediction means, or wherein the at least some digital prediction means are configured externally, in at least one prediction server.

15. The method of claim 14, wherein the data of the at least one patient comprises data from its clinical record.

16. The method of claim 15, wherein the training of the expert module is performed in a centralized or distributed manner.

17. The method of claim 16, wherein the database selection comprises selecting a subset of ICD codes, and filtering them by applying a recursive feature elimination algorithm.

18. The method of claim 17, wherein the database selection comprises tagging the selected codes with a time stamp.

19. The method of claim 16, wherein the variable selection comprises, using the selected codes as input parameters, executing a random forests first model, a naive Bayesian second model, and a linear third model, and determining all the variables of the best model additionally to those that are jointly in the other two models, for selecting the variables with highest predictive capacity.

20. The method of claim 16, wherein the training comprises executing an optimized oropharyngeal dysphagia screening process, CODO, as a function of a random forests first model, and a Bayesian network second model, for training the expert module.

21. The method of claim 16, wherein the prediction comprises executing an optimized oropharyngeal dysphagia screening process, CODO, comprising downloading the latest update of the expert module and using the data of the at least one patient as input to the random forests first model of the expert module and using the data of the at least one patient as input to a Bayesian network second model of the expert module, and determining that there is positive risk of suffering from oropharyngeal dysphagia if the result of both models is above 50%.

22. The method of claim 16, wherein the training comprises executing a random forests first model, and a high information density disperse Bayesian network second model, RBDADI, which comprises Shannon's mutual information index, for training the expert module.

23. The method of claim 16, wherein the prediction comprises downloading the latest expert module update and using the data of the at least one patient as input to the random forests first model of the expert module and using the data of the at least one patient as input to the high information density disperse Bayesian network second model, RBDADI, as a function of Shannon's mutual information index, of the expert module, and determining that there is positive risk of suffering from oropharyngeal dysphagia if the result of both models is above 50%.

24. The method of claim 21 or 23, wherein, if the result of either of both models does not exceed 50%, applying a risk parameter $\lambda$, between 0 and 1, and repeating executing both models and determining that there is positive risk of suffering from oropharyngeal dysphagia if the result of both models is above 50%, or determining that there is no risk of suffering from oropharyngeal dysphagia if the result of both models is equal to or below 50%.

25. A computer program, which comprising instructions which, once executed on a processor, performs the method steps of any one of claims 13 to 24.

26. Computer readable means comprising instructions which, once executed on a processor, performs the method steps of any one of claims 13 to 24.

```
┌─────────────────────┐
│                     │
│        110          │
│                     │
└─────────────────────┘
           │
           ▼
┌─────────────────────┐
│                     │
│        120          │
│                     │
└─────────────────────┘
           │
           ▼
┌─────────────────────┐
│                     │
│        130          │
│                     │
└─────────────────────┘
```

100

**FIG. 1**

FIG. 2

FIG. 3

FIG. 4

EP 4 064 293 A1

EP 4 064 293 A1

**FIG. 5**

**FIG. 6**

EP 4 064 293 A1

700
CODO

**FIG. 7**

EP 4 064 293 A1

800

**FIG. 8**

EP 4 064 293 A1

C

710

920

Y    730    N

D

E

600
RBDADI

**FIG. 9**

**FIG. 10**

1000

RBDADI

FIG. 11

1100
CODO

## INTERNATIONAL SEARCH REPORT

| International application No |
| --- |
| PCT/ES2020/070723 |

**A. CLASSIFICATION OF SUBJECT MATTER**
INV.  G16H50/20      A61B5/00
ADD.  G16H10/60      G16H50/70

According to International Patent Classification (IPC) or to both national classification and IPC

**B. FIELDS SEARCHED**

Minimum documentation searched (classification system followed by classification symbols)
G16H  A61B

Documentation searched other than minimum documentation to the extent that such documents are included in the fields searched

Electronic data base consulted during the international search (name of data base and, where practicable, search terms used)
EPO-Internal, WPI Data

**C. DOCUMENTS CONSIDERED TO BE RELEVANT**

| Category* | Citation of document, with indication, where appropriate, of the relevant passages | Relevant to claim No. |
| --- | --- | --- |
| Y | US 2019/108915 A1 (SPURLOCK III CHARLES FLOYD [US]) 11 April 2019 (2019-04-11) page 1, paragraph 0002 - page 5, paragraph 0056 figures 1-6 ----- -/-- | 1-26 |

| X | Further documents are listed in the continuation of Box C. | | X | See patent family annex. |

\* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 31 March 2021 | 08/04/2021 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Sasa Bastinos, Ana |

Form PCT/ISA/210 (second sheet) (April 2005)

## INTERNATIONAL SEARCH REPORT

International application No

PCT/ES2020/070723

C(Continuation).    DOCUMENTS CONSIDERED TO BE RELEVANT

| Category* | Citation of document, with indication, where appropriate, of the relevant passages | Relevant to claim No. |
|---|---|---|
| Y | KAMKAR IMAN ET AL:  "Stable feature selection for clinical prediction: Exploiting ICD tree structure using Tree-Lasso", JOURNAL OF BIOMEDICAL INFORMATICS, vol. 53, 9 December 2014 (2014-12-09), pages 277-290, XP055788362, US ISSN: 1532-0464, DOI: 10.1016/j.jbi.2014.11.013 abstract page 278, third paragraph in the left column - last paragraph in the right column; figure 1 section "3. Methodologies" on pages 279-281; figure 2 section "4. Experiments", in particular pages 281, 282, 284, and 286-288 section "5. Conclusion" on pages 289-290 ----- | 1-26 |
| A | Anonymous:  "Home Page ICD API", , 24 March 2019 (2019-03-24), XP055788303, Retrieved from the Internet: URL:https://web.archive.org/web/2019032405 1700/https://icd.who.int/icdapi [retrieved on 2021-03-22] the whole document ----- | 1-26 |
| A | Anonymous:  "Feature selection - Wikipedia", , 13 October 2019 (2019-10-13), XP055788078, Retrieved from the Internet: URL:https://en.wikipedia.org/w/index.php?t itle=Feature_selection&oldid=920990787 [retrieved on 2021-03-22] the whole document ----- | 1-26 |
| A | Beyeler ET AL: "Combining Different Algorithms into an Ensemble" In: "Machine Learning for OpenCV", 14 April 2014 (2014-04-14), Packt Publishing, Limited, XP055788960, pages 263-295, page 292 - page 295 ----- | 1-26 |

-/--

Form PCT/ISA/210 (continuation of second sheet) (April 2005)

**INTERNATIONAL SEARCH REPORT**

International application No

PCT/ES2020/070723

| C(Continuation). | DOCUMENTS CONSIDERED TO BE RELEVANT | |
|---|---|---|
| Category* | Citation of document, with indication, where appropriate, of the relevant passages | Relevant to claim No. |
| A | Anonymous: "Centralized computing - Wikipedia", <br> , <br> 18 June 2015 (2015-06-18), XP055654594, <br> Retrieved from the Internet: <br> URL:https://en.wikipedia.org/w/index.php?t <br> itle=Centralized_computing&oldid=667528078 <br> [retrieved on 2019-12-20] <br> the whole document <br> ----- | 1-26 |

Form PCT/ISA/210 (continuation of second sheet) (April 2005)

31

## INTERNATIONAL SEARCH REPORT
Information on patent family members

International application No

PCT/ES2020/070723

| Patent document cited in search report | Publication date | Patent family member(s) | | Publication date |
|---|---|---|---|---|
| US 2019108915 A1 | 11-04-2019 | US | 2019108912 A1 | 11-04-2019 |
| | | US | 2019108915 A1 | 11-04-2019 |
| | | WO | 2019071098 A2 | 11-04-2019 |

Form PCT/ISA/210 (patent family annex) (April 2005)